Europäisches Patentamt

European Patent Office

Office européen des brevets

(19)

(11) Veröffentlichungsnummer: **0 166 107**
**A2**

(12) # EUROPÄISCHE PATENTANMELDUNG

(21) Anmeldenummer: 85104824.9

(22) Anmeldetag: 20.04.85

(51) Int. Cl.⁴: **C 08 K 5/01,** C 08 L 83/00,
A 61 K 6/10

(30) Priorität: 05.05.84 DE 3416694

(71) Anmelder: **BAYER AG, Konzernverwaltung RP
Patentabteilung, D-5090 Leverkusen 1 Bayerwerk (DE)**

(43) Veröffentlichungstag der Anmeldung: **02.01.86
Patentblatt 86/1**

(72) Erfinder: **Schwabe, Peter, Dr., Dudweiler Strasse 17,
D-5090 Leverkusen 1 (DE)**
Erfinder: **Voigt, Reiner, Dr.,
Gustav-Radbruch-Strasse 14, D-5090 Leverkusen 3 (DE)**

(84) Benannte Vertragsstaaten: **AT BE CH DE FR GB IT LI NL
SE**

(54) **Verwendung von Paraffin- oder Mikrowachsen für Silikonpasten, deren Konfektionierung und Verwendung.**

(57) Die vorliegende Erfindung betrifft die Verwendung von Paraffin- oder Mikrowachsen in Silikon-Pasten, insbesondere in knetbaren Massen zur Herstellung genauer Abformungen von Zähnen. Bei den Silikon-Pasten handelt es sich vorzugsweise um an sich bekannte kaltvulkanisierende Zweikomponenten-Silikonkautschuksysteme, bei denen zwei Pasten vermengt werden, die dann nach ca. 2–5 Minuten bei Raumtemperatur vernetzen.

EP 0 166 107 A2

0166107

BAYER AKTIENGESELLSCHAFT        5090 Leverkusen, Bayerwerk
Konzernverwaltung RP
Patentabteilung        Sft/Kü-c

Verwendung von Paraffin- oder Mikrowachsen für Silikon-
pasten, deren Konfektionierung und Verwendung

Die vorliegende Erfindung betrifft die Verwendung von
Paraffin- oder Mikrowachsen in Silikon-Pasten, insbesondere in knetbaren Massen zur Herstellung genauer
Abformungen von Zähnen. Bei den Silikon-Pasten handelt
es sich vorzugsweise um an sich bekannte kaltvulkanisierende Zweikomponenten-Silikonkautschuksysteme,
bei denen zwei Pasten vermengt werden, die dann nach
ca. 2 - 5 Minuten bei Raumtemperatur vernetzen.

Silikon-Pasten sind in der Zahnheilkunde als Abformmassen weit verbreitet. Im allgemeinen bestehen sie aus
einem mit Füllstoffen vermischten Silikonöl auf Basis
eines endständige Hydroxylgruppen aufweisenden Polydimethylsiloxans, welches je nach Applikationsmethode
in verschiedenen Konsistenzen erhältlich ist, und einer
flüssigen oder pastenförmigen Härterkomponente, die
ein Metallsalz einer Monocarbonsäure als Katalysator
und einen Kieselsäureester als Vernetzer enthält.

Le A 22 946 -Ausland

0166107

Diese beiden Komponenten werden vor der Anwendung miteinander gemischt und vernetzen bei Raumtemperatur innerhalb von 2 - 5 Minuten infolge einer Polykondensationsreaktion. Hierbei entstehen neben dem vernetzten Silikongummi noch geringe Mengen Alkohol, die langsam aus dem Gummi heraus diffundieren und eine lineare Schrumpfung hervorrufen, was bei den Abformungen zu Ungenauigkeiten führt.

Wesentlich geringer ist die lineare Schrumpfung bei den erst seit wenigen Jahren bekannten Vinylsilikon-Abformmassen. Diese Massen bestehen aus zwei Pasten: einer Silikonöl, Füllstoff und Vernetzer enthaltenden Basispaste und einer Silikonöl, Füllstoff und Katalysator enthaltenden Katalysatorpaste.

Bei dem Silikonöl handelt es sich hierbei um ein endständige Vinylgruppen aufweisendes Polydimethylsiloxan, der Vernetzer enthält die reaktiven SiH-Gruppen und der Katalysator besteht vorzugsweise aus einem Platinkomplex. Zu der größeren Dimensionsgenauigkeit der Abformung kommt bei diesem System noch die bessere Dosierbarkeit von Basis- und Katalysatorpaste infolge gleicher Pasten-Viskosität und dem abgestimmten Mischungsverhältnis von 1:1 der beiden Pasten hinzu, sowie die völlige Geschmack- und Geruchlosigkeit der Pasten.

Da in der Zahnheilkunde verschiedene Techniken zur Abformung bezahnter, teilbezahnter und unbezahnter Kiefer und der Schleimhaut angewendet werden, ist ein Angebot von Abformmassen in verschiedenen Viskositäts-

**Le A 22 946**

klassen notwendig, z.B. niedrig-, mittel-, hochviskose und knetbare Massen. Diese Massen bestehen jeweils aus einer Basispaste und einer speziell auf die erforderliche Verarbeitungszeit eingestellten Katalysatorpaste.

Während bei den niedrig-, mittel- und hochviskosen Massen die Basis- und die Katalysatorpaste aus Tuben oder Kartuschen in gleichlangen Strängen auf einen Anmischblock ausgedrückt und mit einem Spatel vermischt werden, entnimmt man die Basis- und Katalysatorpaste der knetbaren Abformmasse mit entsprechend gekennzeichneten Dosierlöffeln aus Kunststoffdosen oder -bechern und verknetet die gleichgroßen Klumpen mit den Fingern zu einer homogenen Masse. Diese Masse wird in einen Abformlöffel gegeben und dann im Mund des Patienten plaziert. Nach einigen Minuten kann der Löffel mit der zu Gummi vernetzten Masse aus dem Mund des Patienten entfernt werden. Die negative Form der entsprechenden Kiefersituation wird danach noch durch Einsatz von niedrigviskoser Abformmasse korrigiert und dann mit einem wäßrigen Gipsbrei ausgegossen, der im erhärteten Zustand als Gipsmodell die Kiefersituation wiedergibt.

Bei der Entnahme der Basis- und Katalysatorpasten und auch beim Mischen der Pasten durch Kneten mit den Fingern ist es wichtig, daß die Masse nicht an den Entnahmelöffeln bzw. den Fingern klebt und/oder Rückstände hinterläßt. Das Kleben kann man durch Auswahl geeigneter Füllstoffe und durch Zusatz von Paraffinöl verhindern. Während man bei den kondensationsvernetzenden Silikonabformmassen den Klebeeffekt auch mit Talkum teilweise abdecken kann, ist dies bei den additionsvernetzenden Vinylsilikon-Abformmassen nicht möglich, da Talkum das Abbindeverhalten der Massen negativ beeinflußt. Der Zusatz von Paraffinöl ist auch nicht ganz unproblematisch. Einerseits benötigt man eine Menge von 6-8 Gewichtsprozent in der Masse, um das Kleben zu verhindern, andererseits schwitzen sowohl die Pasten als auch die Abformungen während der Lagerung einen Teil des Paraffinöles aus, d.h. in den Vertiefungen der Pastenoberfläche sammelt sich Paraffinöl an bzw. auf der Oberfläche der Abformungen bilden sich Tropfen von Paraffinöl. Letztere können zum einen den Verbund zwischen der ausgehärteten knetbaren Masse und dem niedrigviskosen Korrekturmaterial verhindern, falls die Korrektur erst nach einem längeren Zeitraum vorgenommen wird, und zum anderen Fehlstellen auf dem Gipsmodell ergeben. Außerdem verringert der Paraffinölzusatz die notwendige Adhäsion der Masse an den Wandungen der Abformlöffel, bzw. am Abformlöffel der mit Adhäsiv behandelt wurde.

Diese Probleme werden erfindungsgemäß durch den Einsatz von Paraffin- oder Mikrowachsen in den Pasten vermieden. Um die Klebrigkeit der Pasten zu eliminieren, benötigt man vorzugsweise nur noch etwa 4-5 Gewichtsprozent Paraffinöl und 1,5 - 5 Gewichtsprozent Paraffin- oder Mikrowachs in der Paste. Die Paraffin- oder Mikrowachse besitzen einen Schmelzbereich zwischen 30-55°C; sie sind also bei Raumtemperatur fest, werden aber durch Energiezufuhr beim Mischen durch Kneten weich. Das Paraffinöl neigt in den erfindungsgemäßen Pasten weniger zum Ausschwitzen. Man erhält auf diese Weise lagerstabilere Massen bzw. Ab-

Le A 22 946

formungen, d.h. es bilden sich kaum noch Tropfen von Paraffinöl in der Paste bzw. auf dem Gummi der Abformung, so daß auch nach längerer Lagerung der Abformung eine einwandfrei Korrekturabformung durchgeführt und/oder ein einwandfreies Gipsmodell hergestellt werden kann.

Außerdem ist die Haftung der Masse an den Wandungen der Abformlöffel verbessert.

Gegenstand der Erfindung ist somit die Verwendung von Paraffin- und/oder Mikrowachsen für pastenförmige Silikonmassen, wobei die Wachse vorzugsweise in einer Menge von 1-10 Gew.-% insbesondere 1,5-5 Gew.-%, bezogen auf die Paste, angewandt werden und vorzugsweise einen Schmelzbereich zwischen 30 und 55°C, insbesondere zwischen 35 und 50°C besitzen.

Gegenstand der Erfindung sind darüber hinaus auch Silikonpasten, insbesondere Silikon-Abformmassen, welche derartige Wachse enthalten.

Gegenstand der Erfindung ist auch die Konfektionierung der Pasten in Portionspackungen, dadurch gekennzeichnet, daß die für eine Abformung benötigte Menge Abformmasse z.B. in eine tiefgezogene Kunststoffolie gefüllt, mit Aluminiumfolie abgedeckt und verschweißt wird.

Vorzugsweise werden die Paraffin- und/oder Mikrowachse in bei Raumtemperatur härtbaren Dentalmassen auf Polysiloxanbasis verwendet. Man unterscheidet dabei, wie schon eingangs ausgeführt, additionsvernetzende und kondensationsvernetzende Systeme. Als wesentliche

Le A 22 946

Komponenten enthalten nach dem Additionssysteme vernetzende erfindungsgemäße Pasten

a) Organopolysiloxane mit zwei oder mehr Vinylgruppen im Molekül

b) anorganische Füllstoffe (unbehandelt oder oberflächenmodifiziert),

c) Organohydrogenpolysiloxane als Vernetzer,

d) einen Katalysator zur Beschleunigung der Additionsreaktion,

e) Paraffinöl

f) Paraffin- oder Mikrowachs und gegebenenfalls

g) Farbstoffe.

Die nach dem Kondensationssystem mit einer flüssigen oder pastenförmigen Härterkomponente, die aus einem Metallsalz einer Monocarbonsäure als Katalysator und einem Kieselsäureester als Vernetzer besteht, vernetzenden erfindungsgemäßen Pasten enthalten als wesentliche Bestandteile

h) Organopolysiloxane mit zwei oder mehr Hydroxylgruppen in Molekül,

i) Füllstoffe,

j) Paraffinöl,

Le A 22 946

k)   Paraffin- oder Mikrowachse und gegebenenfalls

i)   Farbstoffe.

Die erfindungsgemäßen knetbaren Silikonpasten zeichnen sich durch ihre Lagerstabilität und Klebfreiheit beim Vermischen von Basis- und Katalysatorpaste bzw. von Paste und Härterkomponente aus. Sie eignen sich für die Herstellung genauer Abformungen von Zähnen wegen ihrer detailgetreuen Wiedergabe im Gipsmodell, nachdem die Vernetzer- und Katalysatorpaste gut gemischt in die Mundhöhle eingebracht und darin verfestigt wurde, die Abformung mit einer Gipsaufschlämmung ausgegossen und zu einem Modell ausgehärtet worden ist. Dieses gute Ergebnis wird erzielt, weil sich auf der Oberfläche der Abformung keine Tropfen von Paraffinöl bilden, die die Verbindung zwischen der Vorabformung aus den knetbaren Massen und der Korrekturabformung aus niedrigviskosen Massen stören und/oder die Oberfläche des Gipsmodells verfälschen.

Die Einsatzstoffe der oben genannten bei Raumtemperatur härtbaren Pasten sind an sich bekannt.

Bei dem Silikonöl (a) handelt es sich um ein vinylendgestopptes Polydimethylsiloxan, dessen Viskosität bevorzugt im Bereich von 500 bis 5.000.000 mPa.s bei 20°C liegt.

Als Füllstoffe (b) eignen sich z.B. Quarz- und Cristobalitmehle, Calciumsulfat, Diatomeenerde, Talkum und Calciumcarbonat. Die Teilchengröße der Füllstoffe liegt

Le A 22 946

vorzugsweise zwischen 1 und 25 μ. Zu feinteilige Füllstoffe können zu einer unerwünschten Strukturviskosität
der Paste führen.

Der Vernetzer (c) ist ein Polydimethylsiloxan, welches
in seinem Molekül Wasserstoffatome an mindestens zwei
Siliciumatomen aufweist.

Bei dem Katalysator (d) handelt es sich z.B. um einen
Platinkomplex, der aus Hexachloroplatin-(IV)-säure hergestellt wurde. Auch diese Verbindungen sind an sich bekannt.

Das Paraffinöl (e) besteht aus einem bei Raumtemperatur
flüssigen Gemisch von Alkanen mit einer Viskosität von
vorzugsweise 120-300 mPa.s, besonders bevorzugt 170-230
mPa.s, bei 20°C.

Die erfindungsgemäß zu verwendenden Paraffin- oder Mikrowachse (f) sind Gemische von gerad- und verzweigtkettigen Alkanen (bevorzugt von $C_{15}$ bis $C_{40}$) mit einem
Schmelzbereich von vorzugsweise 30 bis 55°C, insbesondere 35-50°C.

Farbstoffe (g) werden bevorzugt zur Unterscheidung der
Basis- und Katalysatorpaste und zur Mischkontrolle eingesetzt. Anorganische und organische Farbpigmente
sind bevorzugt.

Bei dem Silikonöl (h) handelt es sich um ein hydroxyl-

Le A 22 946

endgestopptes Polydimethylsiloxan, dessen Viskosität vorzugsweise im Bereich von 500 bis 200.000 mPa.s bei 20°C liegt.

Die Füllstoffe (i), das Paraffinöl (j), das Paraffin- oder Mikrowachs (k) und die Farbstoffe (l) sind dieselben Substanzen wie oben unter (b), (e), (f) und (g) beschrieben.

Wie eingangs geschildert, kommen die knetbaren Abformmassen in Kunststoffdosen oder -bechern in den Handel. Bei den additionsvernetzenden Vinylsilikonabformmassen werden die Basis- und Katalysatorpaste vor der Anwendung mit entsprechend gekennzeichneten Dosierlöffeln aus den Gefäßen entnommen und die gleichgroßen Klumpen mit den Fingern zu einer homogenen Masse verknetet. Auch die kondensationsvernetzende Silikonabformmasse wird mittels Dosierlöffel aus dem Gefäß entnommen und mit der entsprechenden Menge an pastenförmigem oder flüssigem Härter durch Kneten mit den Fingern vermischt.

Der erfindungsgemäße Zusatz von Paraffin- oder Mikrowachsen kann zu Pasten führen, die einerseits bei Raumtemperatur für eine Entnahme mit einem Dosierlöffel zu fest sind, die andererseits jedoch durch die Energiezufuhr beim Kneten die für die Anwendung günstige weiche, geschmeidige Konsistenz erhalten. Für diese Pasten empfiehlt sich weiterhin erfindungsgemäß die Konfektionierung in Portionspackungen,

Le A 22 946

wobei man die für eine Abformung notwendige Menge an Abformmasse in eine tiefgezogene Kunststoffolie z.B. aus Polystyrol, Polyethylen, Polypropylen u.ä.) abfüllt, mit einer mechanisch leicht zerstörbaren Abdeckung (z.B. einer Aluminiumfolie) versieht und verschweißt. Bei kondensationsvernetzenden Silikonabformmassen wird dabei die für eine Abformung benötigte Menge als jeweils eine Portion abgepackt, vor der Anwendung die Paste durch die Aluminiumfolie gedrückt und mit einer entsprechenden Menge an pastenförmigem Härter aus einer Tube oder flüssigem Härter aus einer Flasche mit den Fingern verknetet. Bei der Vinylsilikonabformmasse wird entsprechend die für eine Abformung benötigte jeweilige Menge an Basis- und Katalysatorpaste verpackt. Vor der Anwendung werden die beiden Pasten durch die Aluminiumfolie gedrückt und mit den Fingern verknetet.

Die nachfolgenden Beispiele, in denen alle Teile Gewichtsteile bedeuten, erläutern die Erfindung.

Le A 22 946

Beispiel 1 (Vergleichsversuch)

Die Basispaste wurde hergestellt durch Vermischen in einem Kneter von 175 Teilen vinylendgestopptem Polydimethylsiloxan mit einer Viskosität von 80.000 mPa.s bei 20°C, 50 Teilen dimethylhydrogensilylendgestopptem Polydimethylsiloxan mit einer Viskosität von 50 mPa.s bei 25°C, 650 Teilen Quarz-Feinstmehl mit einer mittleren Teilchengröße von ca. 4 μ, 60 Teilen Calciumcarbonat mit einer mittleren Teilchengröße von ca. 8 μ, 60 Teilen Paraffinöl mit einer Viskosität von ca. 180 mPa.s bei 20°C und 5 Teilen anorganischem Farbpigment.

Die Herstellung der Katalysatorpaste erfolgte durch Vermischen in einem Kneter von 229,8 Teilen vinylendgestopptem Polydimethylsiloxan mit einer Viskosität von 80.000 mPa.s bei 20°C, 650 Teilen Quarz-Feinstmehl mit einer mittleren Teilchengröße von ca. 4 μ, 60 Teilen Calciumcarbonat mit einer mittleren Teilchengröße von ca. 8 μ, 60 Teilen Paraffinöl mit einer Viskosität von 180 mPa.s und 0,2 Teilen eines Komplexes aus Platin und Divinyltetramethyldisiloxan.

Beide Pasten sind geschmeidig, klebfrei und lassen sich gut kneten, bei einer Lagerung von 7 Tagen bilden sich jedoch in den Vertiefungen der beiden Pastenoberflächen Ansammlungen von Paraffinöl.

15 g Basispaste und 15 g Katalysatorpaste wurden mit den Fingern in 30 Sekunden zu einer homogenen Masse verknetet, auf einen Abformlöffel und unter geeignetem

Le A 22 946

Druck in den Mund gebracht. Innerhalb von 5 Minuten härtete die Masse zu einem Elastomeren aus. Nach Entnahme aus dem Mund, Abwaschen mit fließendem Wasser und Abtupfen mit Zellstoff wurde die Abformung 24 Stunden bei Raumtemperatur gelagert. An der Oberfläche der Abformung hatten sich danach Tröpfchen von Paraffinöl gebildet, welche sich auf das Gipsmodell, hergestellt durch Ausgießen der Abformung mit einer Gipsaufschlämmung von 100 Teilen Calciumsulfat-Hemihydrat und 30 Teilen Wasser und anschließender Lagerung über 30 Minuten, in Form von Vertiefungen übertrugen und somit das Gipsmodell verfälschten.

In eine wie vorbeschrieben hergestellte, 24 Stunden gelagerte und mit Öltropfen versehene Abformung wurde eine niedrigviskose Vinylsilikon-Abformmasse zur Korrekturabformung gegeben und unter geeignetem Druck in den Mund gebracht. Die nach 5 Minuten zu einem Elastomeren ausgehärtete Masse hatte nach dem Entfernen aus dem Mund an den ölbenetzten Stellen keine Haftung zu dem Vorabformmaterial.

Beispiel 2 (erfindungsgemäß)

In einem Kneter wurde die Basispaste hergestellt durch Vermischen von 190 Teilen vinylendgestopptem Polydimethylsiloxan mit einer Viskosität von 80.000 mPa.s bei 20°C, 55 Teilen dimethylhydrogensilyl-endgestoppten Polydimethylsiloxan mit einer Viskosität von 50 mPa.s, 40 Teilen Paraffinöl mit einer Viskosität von 180 mPa.s bei 20°C, 22 Teilen Mikro-

Le A 22 946

wachs mit einem Erstarrungspunkt von 43-45°C nach
DIN 51556, 688 Teilen Quarzfeinstmehl mit einer
mittleren Teilchengröße von ca 4 μ und 5 Teilen
anorganischem Farbpigment.

Die Katalysatorpaste wurde in einem Kneter hergestellt durch Vermischen von 270 Teilen vinylendgestopptem Polydimethylsiloxan mit einer Viskosität von 80.000 mPa.s bei 20°C, 40 Teilen Paraffinöl mit einer Viskosität von 180 mPa.s bei 20°C,
22 Teilen Mikrowachs mit einem Erstarrungspunkt
von 43-45°C nach DIN 51556, 667,8 Teilen Quarzfeinstmehl mit einer mittleren Korngröße von 4 μ
und 0,2 Teilen Platin-Siloxan-Komplex.

Beide Pasten waren klebfrei, wiesen nach einer
Lagerung von 2 Monaten keine Paraffinöl-Ausscheidung
auf und hatten eine so feste Konsistenz, daß die Entnahme aus den Kunststoffdosen mit Dosierlöffeln nur
mit Kraftaufwand möglich war. Ausgehend von der Überlegung, daß für die Abformung eines Kiefers ca.
50 ml Abformmasse notwendig sind, wurde eine 0,4 mm
dicke Folie aus Polypropylen mit den Maßen 25 x 12 cm
durch Tiefziehen mit 2 Reihen à 5 Vertiefungen mit
den Maßen 5 cm Durchmesser und 1,3 cm Tiefe versehen.
In die eine Reihe Vertiefungen wurde die Basispaste,
in die andere die Katalysatorpaste gefüllt, danach
mit Aluminiumfolie abgedeckt und zugeschweißt.

Vor dem Abformen wurde je eine Portion Basis- und
Katalysatorpaste durch die Aluminiumfolie gedrückt

Le A 22 946

und durch Kneten gemischt, wobei die Masse weich und geschmeidig wurde, ohne zu kleben. Die Abformung wies nach 24 Stunden keine Öltröpfchen an der Oberfläche auf. Das Korrekturmaterial haftete einwandfrei und das Gipsmodell war ohne Fehler.

**Beispiel 3** (Vergleichsversuch)

In einem Kneter wurde eine Paste hergestellt durch Vermischen von 210 Teilen hydroxylendgestopptem Polydimethylsiloxan mit einer Viskosität von 50.000 mPa.s bei 20°C, 80 Teilen Paraffinöl mit einer Viskosität von 180 mPa.s bei 20°C, 100 Teilen Calciumcarbonat, 600 Teilen Quarzfeinstmehl von Beispiel 1 und 10 Teilen Titandioxid.

Die Paste war geschmeidig, klebfrei und ließ sich gut kneten. Bei einer Lagerung von 7 Tagen bildeten sich jedoch in den Vertiefungen der Pastenoberfläche Ansammlungen von Paraffinöl.

25 g Paste und 1 g einer Härterkomponente, bestehend aus Dibutylzinndilaurat und Tetraethoxysilan wurden in 30 Sekunden zu einer homogenen Masse verknetet, auf einen Abformlöffel und unter geeignetem Druck in den Mund gebracht. Innerhalb von 5 Minuten härtete die Masse zu einem Elastomeren aus. Nach Entnahme aus dem Mund, Abwaschen mit fließendem Wasser und Abtupfen mit Zellstoff wurde die Abformung 24 Stunden bei Raumtemperatur gelagert. An der Oberfläche der Abformung hatten sich danach Tröpfchen von Paraffinöl gebildet, welche sich auf das Gipsmodell, hergestellt durch Ausgießen der Abformung

Le A 22 946

mit einer Gipsaufschlämmung von 100 Teilen Calciumsulfat-Hemihydrat und 30 Teilen Wasser und anschließender Lagerung über 30 Minuten, in Form von Vertiefungen übertrugen und somit das Gipsmodell verfälschten.

In eine wie oben beschrieben hergestellte, 24 Stunden gelagerte und mit Öltropfen versehene Abformung wurde eine niedrigviskose Silikon-Abformmasse zur Korrekturabformung gegeben und unter geeignetem Druck in den Mund gebracht. Die nach 5 Minuten zu einem Elastomeren ausgehärtete Masse hatte nach dem Entfernen aus dem Mund an den ölbenetzten Stellen keine Haftung zu dem Vorabformmaterial.

## Beispiel 4 (erfindungsgemäß)

Eine Paste wurde in einem Kneter hergestellt durch Vermischen von 220 Teilen hydroxylendgestopptem Polydimethylsiloxan mit einer Viskosität von 50.000 mPa.s bei 20°C, 40 Teilen Paraffinöl mit einer Viskosität von 180 mPa.s, 30 Teilen Mikrowachs mit einem Erstarrungspunkt von 38-40°C nach DIN 51556, 700 Teilen Quarzfeinstmehl und 10 Teilen Titandioxid.

Die Paste war klebfrei, wies nach einer Lagerung von 2 Monaten keine Paraffinöl-Ausscheidung auf und hatte eine etwas feste Konsistenz. Obwohl sie sich mittels Dosierlöffel aus dem Kunststoffbecher entnehmen ließ, wurde sie zwecks bequemerer Handhabung in Portionen konfektioniert: In eine 0,4 mm dicke Folie aus Poly-

Le A 22 946

propylen mit den Maßen 30 x 6 cm wurden 5 Vertiefungen mit den Maßen 5 x 5 x 2 cm tiefgezogen, in welche die Paste gefüllt, mit Aluminiumfolie abgedeckt und zugeschweißt wurde.

Vor dem Abformen wurde eine Portion durch die Aluminiumfolie gedrückt. Beim Mischen mit der Härterkomponente aus Dibutylzinndilaurat und Tetraethoxysilan wurde die Paste weich und geschmeidig ohne zu kleben. Die Abformung besaß nach 24 Stunden an der Oberfläche keine Öltröpfchen. Das Korrekturmaterial haftete einwandfrei und das Gipsmodell wies keine Fehler auf.

Le A 22 046

## Patentansprüche

1. Verwendung von Paraffin- und/oder Mikrowachs in pastenförmigen Silikonmassen.

2. Verwendung nach Anspruch 1, dadurch gekennzeichnet, daß die Wachse einen Schmelzbereich zwischen 30 und 55°C, vorzugsweise zwischen 35 und 50°C, besitzen.

3. Verwendung von Paraffin- und/oder Mikrowachs nach Anspruch 1 oder 2 in knetbaren, bei Raumtemperatur aushärtenden Dental-Abformmassen auf Silikonbasis.

4. Verwendung gemäß Anspruch 1 bis 3 in additionsvernetzenden Systemen.

5. Verwendung gemäß Anspruch 1 bis 3 in kondensationsvernetzenden Systemen.

6. Pastenförmige Silikonmasse, insbesondere knetbare Abformmasse, dadurch gekennzeichnet, daß sie Paraffin- und/oder Mikrowachs enthält.

7. Masse nach Anspruch 6, dadurch gekennzeichnet, daß das Wachs einen Schmelzbereich zwischen 30 und 55°C, vorzugsweise zwischen 35 und 50°C, aufweist.

Le A 22 946

8. Masse nach Anspruch 6 oder 7, dadurch gekennzeichnet, daß sie 1-10 Gew.-%, vorzugsweise 1,5-5 Gew.-%, des Wachses enthält.

9. Masse nach Anspruch 6 bis 8, dadurch gekennzeichnet, daß sie zusätzlich 3-5 Gew.-% Paraffinöl enthält.

10. Konfektionierte Masse nach Anspruch 6 bis 9, dadurch gekennzeichnet, daß sie portionsweise z.B. in einer tiefgezogenen Kunststoffolie abgepackt ist, welche mit einer Abdeckfolie versehen ist.

Le A 22 946